# EUROPÄISCHE PATENTSCHRIFT

(11) **EP 3 981 370 B1**
(45) Veröffentlichungstag und Bekanntmachung des Hinweises auf die Patenterteilung: **25.09.2024**
(21) Anmeldenummer: 21201339.5
(22) Anmeldetag: 07.10.2021
(51) Int. Cl.: A61F 13/06, A61F 13/10, A61F 5/01

(54) **BANDAGE, INSBESONDERE AUSGEBILDET ALS KNIE- ODER ELLBOGENSCHÜTZER**
BANDAGE, IN PARTICULAR DESIGNED AS A KNEE OR ELBOW PROTECTOR
BANDAGE, EN PARTICULIER CONÇU SOUS LA FORME DE PROTÈGE-GENOU OU DE COUDIÈRE

(30) Priorität: 09.10.2020 DE 102020126532
(43) Veröffentlichungstag der Anmeldung: 13.04.2022
(73) Patentinhaber: MPR GmbH & Co. KG, 95615 Marktredwitz (DE)
(72) Erfinder: Pürner, Marcus, 96516 Marktredwitz (DE)
(74) Vertreter: Friderichs, Gunther

(56) Entgegenhaltungen:
- WO-A1-2018/025238
- WO-A1-2018/186803
- IT-A1- MI20 111 636
- KR-A- 20190 120 999
- US-A1- 2010 106 070
- US-A1- 2016 324 675
- US-A1- 2019 175 378
- US-B1- 9 302 137
- US-B2- 7 708 708

## Beschreibung

### Gebiet der Erfindung

Die Erfindung betrifft eine Bandage, welche insbesondere als Gelenkschützer, beispielsweise zum Knie- oder Ellbogenschutz von Sportlern ausgebildet ist.

### Hintergrund der Erfindung

Im Sportbereich werden Bandagen verwendet, beispielsweise um Gelenke zu stabilisieren. Es handelt sich dabei um elastische Schlaufen, die um das Gelenk herumgelegt werden können und die zumindest eine stützende Wirkung haben.

Weiter gibt es Protektoren, die ebenfalls als elastische Schlaufe ausgebildet sind und zusätzlich einen Einsatz aus stoßabsorbierendem Schaumstoff, der zumeist mit einer harten Schale kombiniert ist, aufweist.

Aus der Praxis bekannte Protektoren können den Nachteil haben, dass sie die Haut des Benutzers einschnüren. Dadurch kann insbesondere der Fluss von Lymphflüssigkeit behindert werden.

Die Lymphbahnen sind feinere Blutgefäße als die Kanäle, entlang welcher Lymphflüssigkeit von den obersten Hautschichten zu den Lymphknoten geführt wird, wo die Lymphflüssigkeit gefiltert wird und von wo aus Giftstoffe weiter an Leber und Niere transportiert und dort verarbeitet und abgeschieden werden.

Der Stau von Lymphflüssigkeit kann Schwellungen oder sogar Beschwerden in Form von Schmerzen nach sich ziehen. WO2018025238 offenbart zum Beispiel einen tragbaren Textilartikel zur Unterstützung der Muskelfunktion.

### Aufgabe der Erfindung

Der Erfindung liegt demgegenüber die Aufgabe zugrunde, eine Bandage, insbesondere einen Protektor, bereit zu stellen, bei dem die genannten Nachteile des Standes der Technik zumindest reduziert sind. Insbesondere soll durch die Bandage der Transport von Lymphflüssigkeit gefördert werden.

### Zusammenfassung der Erfindung

Die Aufgabe der Erfindung wird bereits durch eine Bandage, welche insbesondere als Protektor ausgebildet ist, nach Anspruch 1 gelöst.

Bevorzugte Ausführungsformen und Weiterbildungen der Erfindung sind dem Gegenstand der abhängigen Ansprüche, der Beschreibung sowie den Zeichnungen zu entnehmen.

Die Erfindung betrifft eine Bandage. Die Bandage ist insbesondere als Protektor ausgebildet.

Insbesondere ist die Bandage als Knie- oder Ellenbogenschützer ausgebildet.

Die Bandage eine Extremität des Körpers, insbesondere um ein Gelenk herum anlegbar.

Gemäß der Erfindung umfasst die Bandage auf einer an der Extremität anliegenden Seite, also der Innenseite der Bandage, eine Vielzahl elastischer Streifen, die vorzugsweise derart angeordnet sind, dass sie den Lymphbahnen des Trägers der Bandage folgen.

Die Lymphbahnen sind beim Menschen von Mensch zu Mensch nahezu identisch ausgebildet. Insbesondere ist die Richtung der Lymphbahnen in der Regel an der Ausrichtung der Härchen auf der Haut erkennbar.

So gehen die Lymphbahnen von einem Gelenk zunächst sternförmig nach außen, um sodann nach oben und unten umgelenkt zu werden und der Extremität zu folgen.

Der Erfindung liegt die Erkenntnis zugrunde, dass derart angeordnete elastische Streifen aus einem weichen elastischen Material auf der Haut des Trägers haften und so zu einem Zusammenziehen von Hautabschnitten führen.

Die elastischen Streifen sind voneinander beabstandet, insbesondere beträgt der Abstand im Mittel zwischen 0,5 und 2 cm. In den Zwischenräumen zwischen den elastischen Streifen bilden sich Kanäle, durch die Lymphflüssigkeit verbessert fließen kann.

Die elastischen Streifen können insbesondere auf die Innenseite der Bandage aufgedruckt sein.

Vorzugsweise bestehen die elastischen Streifen aus einem Silikon, einem Elastomer oder einem Polyolefin, insbesondere einem Polyurethan.

Die elastischen Streifen bestehen vorzugsweise aus einem weichen Material mit einer Härte nach Shore A (nach DIN ISO 7619-1:2012-2) von über 10, vorzugsweise über 15 und besonders bevorzugt über 20.

Vorzugsweise ist das Material weich, insbesondere beträgt die Härte nach Shore A unter 50, vorzugsweise unter 40, besonders bevorzugt unter 35 und ganz besonders bevorzugt unter 30.

Insbesondere beträgt die Härte Shore A 25 +/- 10, bevorzugt 25 +/- 5.

Ein derartiges weiches Material führt zu einem Klebeeffekt auf der Haut, der das Zusammenziehen von Hautabschnitten verbessert.

Gemäß einer Weiterbildung der Erfindung weisen die elastischen Streifen Unterbrechungen auf, insbesondere in einem Abstand von 0,5 bis 5 cm. Es hat sich herausgestellt, dass durch derartige Unterbrechungen ein störendes Ziehen an den Härchen der Haut weitgehend vermieden werden kann.

Soweit die Bandage als Gelenkschützer ausgebildet ist, können sich die elastischen Streifen oberhalb des Gelenks auch schneiden und dort insbesondere dachförmige Abschnitte bilden.

Vorzugsweise sind auf der Bandage zumindest abschnittsweise 3 bis 15, vorzugsweise 6 bis 10 nebeneinander liegende elastische Streifen angeordnet.

Die elastischen Streifen haben gemäß einer Ausführungsform der Erfindung eine Breite von 0,5 bis 2 cm, vorzugsweise von 0,8 bis 1,8 cm.

Die elastischen Streifen stehen vorzugsweise aus dem Trägermaterial hervor.

Insbesondere haben die elastischen Streifen eine Höhe von 0,5 bis 3 mm, vorzugsweise von 0,1 bis 1 mm.

Bei einer Weiterbildung der Erfindung weisen die Streifen unterschiedliche Höhen auf. Insbesondere kann sich die Höhe der Streifen um zumindest 20%, bevorzugt zumindest 30 % unterscheiden.

Gemäß einer Ausführungsform der Erfindung weisen die Streifen untereinander jeweils unterschiedliche Höhen auf.

Gemäß einer weiteren Ausführungsform ändert sich die Höhe zumindest eines Streifens, vorzugsweise einer Vielzahl von Streifen über dessen/deren Länge. Dies kann insbesondere bei einem in Segmente unterteilten Streifen durch unterschiedlich hohe Segmente realisiert sein.

Diese Ausführungsformen können auch miteinander kombiniert sein.

Bei einer Ausführungsform der Erfindung reduziert sich die Höhe von einer Mitte zu einem Rand hin.

Gemäß einer anderen Ausführungsform kann sich die Höhe von einer Mitte zum Rand hin auch vergrößern

Gemäß einer weiteren Ausführungsform wechseln sich höhere und niedrigere Streifen ab.

Über unterschiedliche Höhen in verschiedenen Bereichen können gezielt die Lymphen angeregt werden.

Der Träger ist vorzugsweise als elastisches Flächengebilde ausgebildet.

Insbesondere kann der Träger zumindest abschnittsweise als elastisches Netzgewebe ausgebildet sein. Dieses ist atmungsaktiv, wodurch ebenfalls der Stau von Lymphflüssigkeit reduziert wird.

Gemäß einer Ausführungsform der Erfindung ist der Träger auf einer Rückseite bereichsweise aus einem anderen Material ausgebildet ist. Insbesondere besteht ein mittiger Bereich aus einem Material, welches weniger Zug ausübt.

So wird die Knie-, bzw. Ellenbogenkehle des Trägers nicht mit Zug belastet, was das Tragen angenehmer macht die empfindlichen Kehlenbereiche schont.

Dabei kann z.B. ein Netzmaterial mit geringerer Elastizität als das restliche Material des Trägers verwendet werden.

Der Zug wird überwiegend von dem Trägermaterial oberhalb und unterhalb dieses Bereichs aufgefangen.

Bei einer weiteren Ausführungsform der Erfindung ist der Träger im Kehlenbereich ausgespart.

Bei einer Weiterbildung der Erfindung umfasst die Bandage ein ringförmiges elastisches Band.

Dieses kann insbesondere an einem oberen Ende der Bandage angeordnet sein und das Anziehen der Bandage erleichtern.

Weiter kann die Bandage, soweit diese als Protektor ausgebildet ist, einen Protektoreneinsatz umfassen, welcher von einer stoßabsorbierenden Polsterung umgeben ist.

Die Erfindung kann weiter durch eine Bandage definiert werden, welche ein oder mehrerer vorstehend beschriebener Merkmale ausweisen kann.

Die Bandage ist, insbesondere ein Gelenk herum anlegbar, wodurch ein unteres und ein oberes Ende der Bandage definiert wird.

Gemäß der Erfindung weist die Bandage auf einer an der Extremität anliegenden Seite eine Vielzahl elastischer Streifen auf, wobei eine Vielzahl unterer elastischer Streifen von einem Mittelbereich ausgehend sich im Wesentlichen parallel nach unten erstrecken und wobei eine Vielzahl oberer elastischer Streifen sich schräg nach oben erstrecken.

Vorzugsweise kreuzen sich obere elastische Streifen.

Der Mittelbereich ist erfindungsgemäß von elastischen Streifen ausgespart.

Der Mittelbereich ist rundlich, insbesondere kreisförmig, oval oder birnenförmig ausgestaltet sein.

Der Mittelbereit kann zumindest bereichsweise von einem elastischen Streifen umgeben sein.

### Kurzbeschreibung der Zeichnungen

Der Gegenstand der Erfindung soll, im Folgenden Bezug nehmen auf ein Ausführungsbeispiel anhand der Zeichnungen Fig. 1 bis Fig. 8, näher erläutert werden.
Fig. 1 ist eine Seitenansicht einer erfindungsgemäßen Bandage, welche als Protektor ausgebildet ist.
Fig. 2 zeigt die auf dem Trägermaterial des Protektors angeordneten elastischen Streifen.
Fig. 3 zeigt die Anordnung der elastischen Streifen auf der gegenüberliegenden linken Seite eines korrespondierenden Protektors.
Fig. 4 bis Fig. 6 sind schematische Schnittansichten der Bandage entlang der Linie A-A der Fig. 3.
Fig. 7 und Fig. 8 sind schematische Längsschnitte entlang eines Streifens.

### Detaillierte Beschreibung der Zeichnung

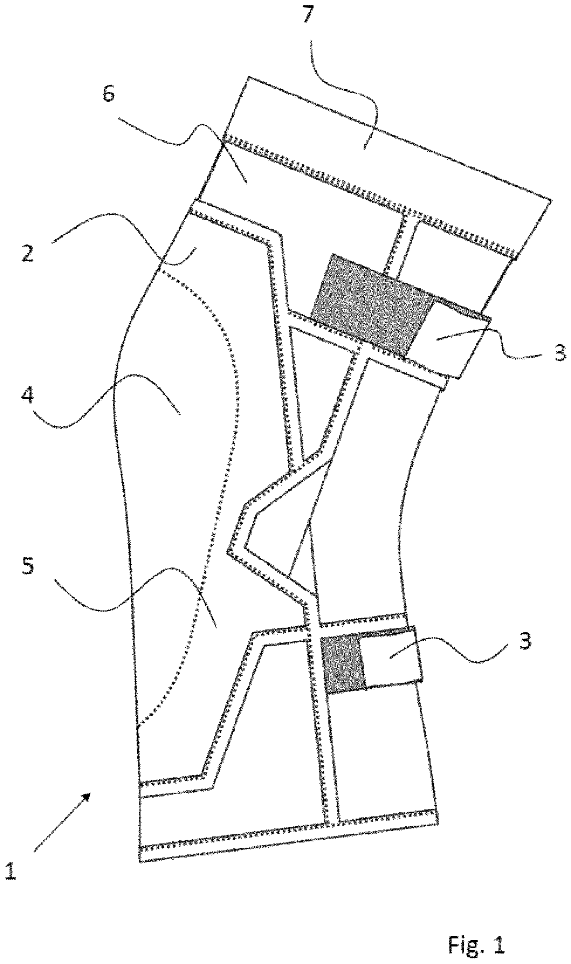

Fig. 1 zeigt in einer Seitenansicht eine Bandage, welche als Protektor 1 ausgebildet ist.

Es handelt sich dabei um den Protektor 1 für das rechte Bein. Der Protektor für das linke Bein (nicht dargestellt) ist spiegelsymmetrisch ausgebildet.

Der Protektor 1 umfasst einen schlauchförmigen Träger 2 aus einem elastischen Material. Es kann sich dabei insbesondere um elastisches Netzgewebe handeln.

Der Protektor 1 ist als Knieschützer ausgebildet. Ein erfindungsgemäßer Ellbogenschützer ist, bis auf die Abmessungen, im Wesentlichen identisch aufgebaut.

Der Protektor 1 kann über die voneinander beabstandeten Klettverschlüsse 3 nach dem Anziehen befestigt werden.

Der Protektor 1 umfasst einen Protektoreneinsatz 4, welcher eine Schale aus rigidem Material (nicht dargestellt) umfassen kann.

Der Protektoreneinsatz 4 ist von einem stoßabsorbierenden elastischen Material in Form einer Polsterung 5 umgeben. So wird durch den Protektoreinsatz 4 das Gelenk bei Stürzen geschützt.

Weiter ist bei diese Ausführungsform im oberen Bereich ein 3D-Netzgewebe 6 vorgesehen. Das 3D-Netzgewebe sorgt für eine im Wesentlichen sich selbst spannende runde Form und erleichtert so das Anziehen.

Ebenso wird das Anziehen durch ein am oberen Ende des Protektors 1 angeordnetes elastisches Band 7 erleichtert.

Auf der Innenseite ist der Träger 2 angrenzend zum Protektoreneinsatz 4 mit elastischen Streifen versehen, welche den Abtransport von Lymphflüssigkeit verbessern.

Im Kehlenbereich 16 ist der Träger 2 aus einem Material ausgebildet, welches weniger Zug auf die Haut des Trägers ausübt als Material im Bereich oberhalb und unterhalb des Kehlenbereichs 16.

Zug auf die Haut des Trägers üben insbesondere die Bereiche mit den Klettverschlüssen 3 aus.

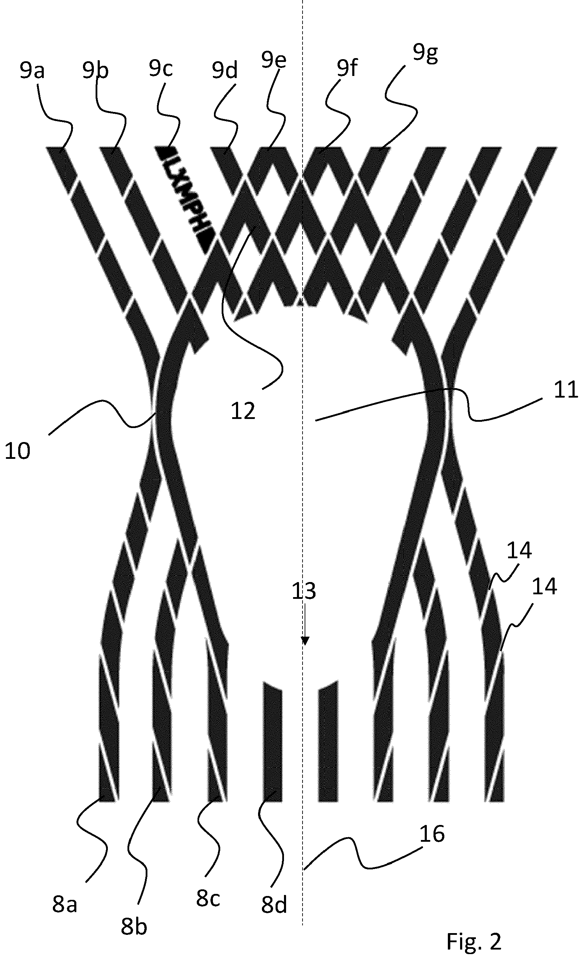

Die Anordnung dieser elastischen Streifen 8a bis 8d, 9a bis 9g, 10 ist in Fig. 2 dargestellt.

Im Bereich des Gelenks ist bei dieser Ausführungsform der Erfindung ein ausgesparter Bereich 11, also ein Mittelbereich, vorhanden, in welchem keine elastischen Streifen vorhanden sind.

Der ausgesparte Bereich 11 ist in etwas birnenförmig ausgebildet und verjüngt sich nach unten. Der ausgesparte Bereich 11 umgibt insbesondere den Bereich der Kniescheibe oder den Bereich des Ellenbogengelenks.

Um den ausgesparten Bereich herum erstrecken sich nach unten elastische Streifen 8a bis 8d.

Die elastischen Streifen 8a und 8b erstrecken sich zunächst seitlich nach außen, um dann nach unten entlang der Extremität weiterzulaufen.

Der äußerste Streifen 8a erstreckt sich bis zu einem elastischen Streifen 10, welcher den ausgesparten Bereich 11 umgibt und geht dann in einen elastischen Streifen 9a über, welche schräg nach oben läuft.

Die oben an dem ausgesparten Bereich 11 angrenzenden elastischen Streifen 9a bis 9g verlaufen gegeneinander gekreuzt in zwei Richtungen nach oben, insbesondere in einem Winkel von 30° bis 80°.

In einem mittleren Bereich überschneiden sich die oberen elastischen Streifen 9e bis 9g und bilden eine dachförmige Anordnung 12.

Eine den ausgesparten Bereich 11 umgebender elastischer Streifen 10 geht in den schräg nach oben laufenden elastischen Streifen 9e sowie in den geradlinig nach unten laufenden elastischen Streifen 8c über.

Der elastische Streifen 8d läuft geradlinig nach unten.

Im unteren Bereich 13 des ausgesparten Bereichs 11 ist der ausgesparte Bereich nicht von dem elastischen Streifen 10 umgeben.

Die elastischen Streifen bestehen aus einem weichen Material, wodurch die Haut zusammengezogen wird und in den Zwischenräumen zwischen den Streifen Kanäle zum Transport von Lymphflüssigkeit geschaffen werden.

Weiter ist zumindest ein Teil der elastischen Streifen mit Unterbrechungen 14 versehen.

Die Unterbrechungen 14 verlaufen in diesem Ausführungsbeispiel ringförmig entgegengesetzt der Orientierung der elastischen Streifen.

Die Anordnung der elastischen Streifen ist in diesem Ausführungsbeispiel im Wesentlichen spiegelsymmetrisch zur Mittellinie 16, so dass obige Ausführung im Hinblick auf die Streifen 8a bis 8d und 9a bis 9g auch für die korrespondierenden gegenüberliegenden elastischen Streifen dienen.

Die elastischen Streifen sind aus Silikon auf den Träger 2 aufgedruckt.

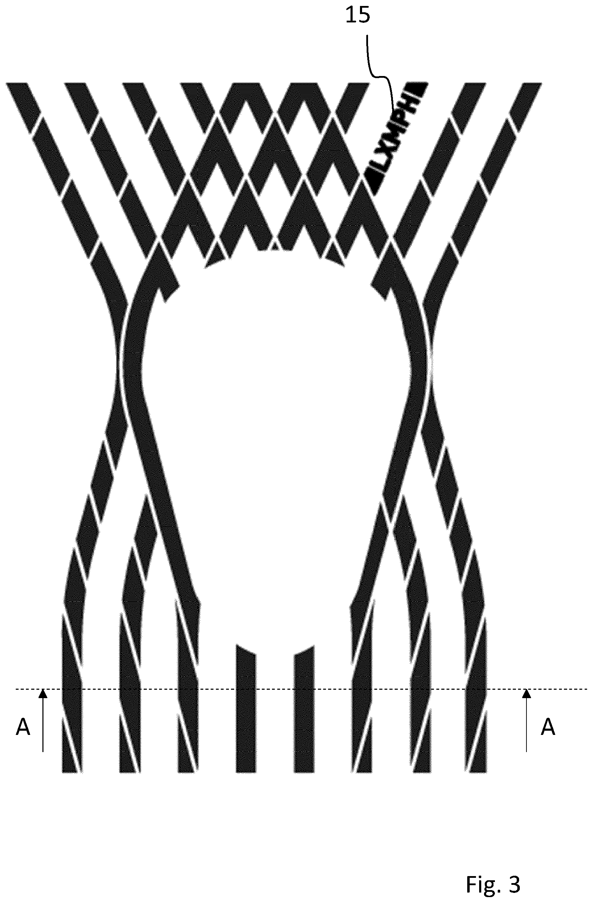

Fig. 3 zeigt das Streifenmuster für den korrespondierend gegenüberliegenden Protektor, also beispielsweise für die linke Seite.

Zu erkennen ist, dass bis auf einen Aufdruck 15, welcher lediglich der Kennzeichnung dient, die Anordnung gegenüber Fig. 2 identisch ist.

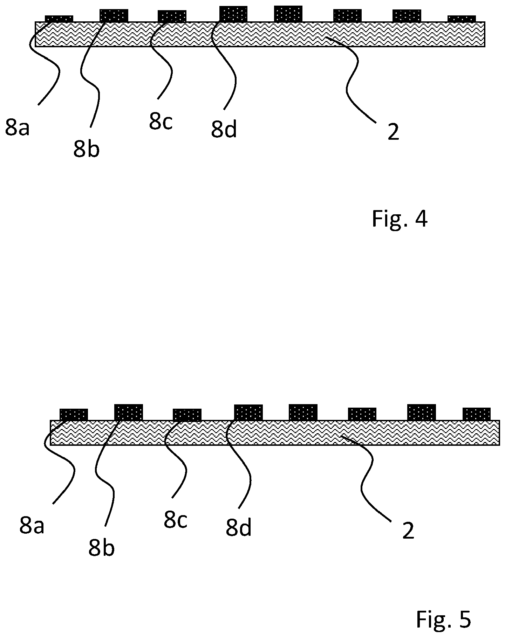

Fig. 4 und Fig. 5 sind schematische Schnittansichten der Bandage entlang der Linie A-A der Fig. 3.

Fig. 4 zeigt eine Ausführungsform der Erfindung, bei welcher sich die Höhe der Streifen ausgehend von einem mittigen Bereich (8d) bis zum Rand (8a) sukzessive verringert.

Fig. 5 zeigt eine Ausführungsform der Erfindung, bei welcher sich hohe (8b, 8d) und niedrige (8a, 8c) Streifen abwechseln.

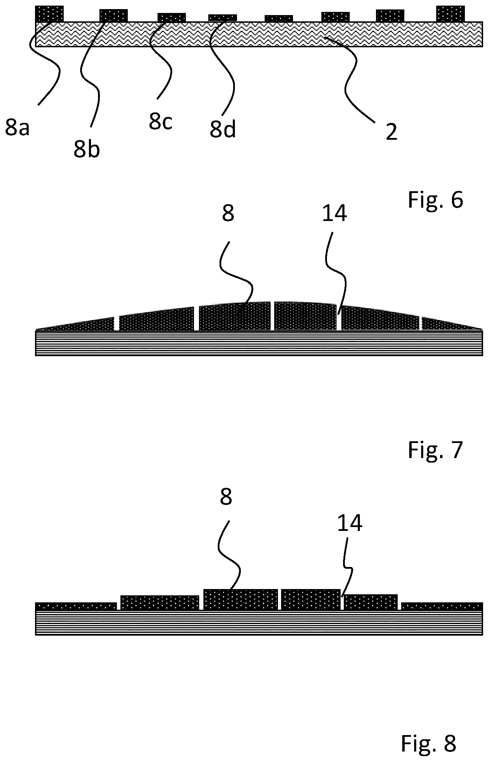

Fig. 6 zeigt eine entsprechende Ausführungsform, bei welcher die Streifen nach außen dicker werden.

Fig. 7 ist ein schematischer Längsschnitt eines Streifens 8, dessen Höhe sich entlang seiner Längserstreckung ändert.

In diesem Ausführungsbeispiel wird der Streifen 8 von einem mittigen Bereich nach außen dünner.

Wie in Fig. 8 dargestellt, können auch die Segmente des Streifens 8, die durch die Unterbrechungen 14 gebildet werden, eine von innen nach außen fallende Höhe aufweisen (oder von außen nach innen, nicht dargestellt).

Auch kann ein Streifen ein wellenartiges Höhenprofil und/oder abwechselnd Segmente mit sich reduzierender und vergrößernder Höhe aufweisen (nicht dargestellt).

Durch die Erfindung konnte einer Bandage, insbesondere ein Protektor bereitgestellt werden, welcher einen verbesserten Abtransport von Lymphflüssigkeit sicherstellt.

### Bezugszeichenliste:

1 Bandage/Protektor
2 Träger
3 Klettverschluss
4 Einsatz (Protektor)
5 Polsterung
6 3d-Netzgewebe
7 elastisches Band
8a-8d elastischer Streifen
9a-9f elastischer Streifen
10 elastischer Streifen
11 ausgesparter Bereich/Mittelbereich
12 dachförmiger Abschnitt
13 unterer Bereich
14 Unterbrechung
15 Aufdruck
16 Kehlenbereich

## Patentansprüche

1. Bandage (1), insbesondere ausgebildet als Protektor (1), wobei die Bandage (1) um eine Extremität, nämlich ein Gelenk, herum anlegbar ist, **dadurch gekennzeichnet, dass** die Bandage (1) auf einer an der Extremität anliegenden Seite eine Vielzahl elastischer Streifen (8a-8d) aufweist, wobei sich elastische Streifen (8a-8d) seitlich vom Gelenk ausgehend erst seitlich erstrecken und dann nach unten umgelenkt werden und wobei sich weitere elastische Streifen (8a-8d) nach oben schräg zu den Seiten erstrecken, und wobei ein rundlich ausgebildeter Mittelbereich (11) von elastischen Streifen (8a-8d) ausgespart (11) ist.

2. Bandage (1) nach dem vorstehenden Anspruch, **dadurch gekennzeichnet, dass** die elastischen Streifen (8a-8d) auf die Innenseite der Bandage (1) aufgedruckt (15) sind.

3. Bandage (1) nach einem der vorstehenden Ansprüche, **dadurch gekennzeichnet, dass** die elastischen Streifen (8a-8d) aus einem Silikon, einem Elastomer oder einem Polyolefin, insbesondere einem Polyurethan, bestehen.

4. Bandage (1) nach einem der vorstehenden Ansprüche, **dadurch gekennzeichnet, dass** die elastischen Streifen (8a-8d) aus einem Material mit einer Härte nach Shore A (nach DIN ISO 7619-1:2012-2) von über 10, vorzugsweise über 15, besonders bevorzugt von über 20 und/oder unter 50, vorzugsweise unter 40, besonders bevorzugt unter 35, ganz besonders bevorzugt unter 30, bestehen.

5. Bandage (1) nach einem der vorstehenden Ansprüche, **dadurch gekennzeichnet, dass** die elastischen Streifen (8a-8d) Unterbrechungen (14) aufweisen, insbesondere in einem Abstand von 0,5 bis 5 cm
und/oder, dass sich die weiteren elastischen Streifen (8a-8d) teilweise schneiden.

6. Bandage (1) nach einem der vorstehenden Ansprüche, **dadurch gekennzeichnet, dass** die Bandage (1) zumindest abschnittsweise 3 bis 15, vorzugsweise 6 bis 10, nebeneinander liegenden elastische Steifen (8a-8d) aufweist
und/oder dass die elastischen Streifen (8a-8d) eine Breite von 0,5 bis 2 cm, vorzugsweise von 0,8 bis 1,8 cm, aufweisen,
und/oder, dass die elastischen Streifen (8a-8d) eine Höhe von 0,05 bis 3 mm, vorzugsweise von 0,1 bis 1 mm, besonders bevorzugt von 0,4 bis 0,8 mm, aufweisen.

7. Bandage (1) nach einem der vorstehenden Ansprüche, **dadurch gekennzeichnet, dass** der Träger (2) als elastisches textiles Flächengebilde, insbesondere als elastisches Netzgewebe, ausgebildet ist.

8. Bandage (1) nach einem der vorstehenden Ansprüche, **dadurch gekennzeichnet, dass** die Bandage (1) als Knie- oder Ellenbogenschützer ausgebildet ist.

9. Bandage (1) nach einem der vorstehenden Ansprüche, **dadurch gekennzeichnet, dass** die Bandage (1) ein randseitiges ringförmiges elastisches Band (7) umfasst.

10. Bandage (1) nach einem der vorstehenden Ansprüche,
**dadurch gekennzeichnet, dass** die Bandage (1) einen Protektoreneinsatz umfasst, welcher von einer Polsterung umgeben ist.

11. Bandage (1) nach einem der vorstehenden Ansprüche,
**dadurch gekennzeichnet, dass** der Träger (2) auf einer Rückseite bereichsweise aus einem anderen Material ausgebildet ist, insbesondere ein Material, das weniger Zug ausübt.

12. Bandage (1) nach einem der vorstehenden Ansprüche,
**dadurch gekennzeichnet, dass** die Streifen eine unterschiedliche Höhe aufweisen, insbesondere, dass sich die Höhe von einer Mitte zu einem Rand reduziert oder dass sich höhere und niedrigere Streifen abwechseln.

13. Bandage (1) nach einem der vorstehenden Ansprüche, wobei die Bandage (1) um eine Extremität, insbesondere ein Gelenk, herum anlegbar ist, wodurch ein unteres und ein oberes Ende der Bandage (1) definiert wird, **dadurch gekennzeichnet, dass** die Bandage (1) auf einer an der Extremität anliegenden Seite eine Vielzahl elastischer Streifen (8a-8d) aufweist, wobei eine Vielzahl unterer elastischer Streifen (8a-8d) von einem Mittelbereich (11) ausgehend sich im Wesentlichen parallel nach unten erstrecken und wobei eine Vielzahl oberer elastischer Streifen (8a-8d) sich schräg nach oben erstrecken.

14. Bandage(1) nach dem vorstehenden Anspruch, **dadurch gekennzeichnet, dass** sich obere elastische Streifen (8a-8d) kreuzen.

15. Bandage (1) nach einem der vorstehenden Ansprüche,
**dadurch gekennzeichnet, dass** das Mittelbereich (11) von elastischen Streifen (8a-8d) ausgespart ist.

## Claims

1. A bandage (1), in particular designed as protector (1),
wherein the bandage (1) can be applied around an extremity, namely a joint, **characterized in that** the bandage (1) has a plurality of elastic strips (8a-8d) on a side applied to the extremity, wherein elastic strips (8a-8d) first extend laterally, starting laterally at the joint, and are then redirected downwards, and wherein further elastic strips (8a-8d) extend upwards obliquely to the sides, and wherein elastic strips (8a-8d) are omitted in a central region (11), which is designed to be roundish.

2. The bandage (1) according to the preceding claim, **characterized in that** the elastic strips (8a-8d) are printed (15) on the inner side of the bandage (1).

3. The bandage (1) according to any one of the preceding claims, **characterized in that** the elastic strips (8a-8d) consist of a silicon, an elastomer or a polyolefin, in particular a polyurethane.

4. The bandage (1) according to any one of the preceding claims, **characterized in that** the elastic strips (8a-8d) consist of a material with a Shore A hardness (according to DIN ISO 7619-1:2012-2) of over 10, preferably over 15, particularly preferably of over 20 and/or below 50, preferably below 40, particularly preferably below 35, most preferably below 30.

5. The bandage (1) according to any one of the preceding claims, **characterized in that** the elastic strips (8a-8d) have recesses (14), in particular at a distance of 0.5 to 5 cm and/or that the further elastic strips (8a-8d) partly intersect.

6. The bandage (1) according to any one of the preceding claims, **characterized in that**, at least in sections, the bandage (1) has 3 to 15, preferably 6 to 10, elastic strips (8a-8d) lying next to one another
and/or that the elastic strips (8a-8d) have a width of 0.5 to 2 cm, preferably of 0.8 to 1.8 cm,
and/or that the elastic strips (8a-8d) have a height of 0.05 to 3 mm, preferably of 0.1 to 1 mm, particularly preferably of 0.4 to 0.8 mm.

7. The bandage (1) according to any one of the preceding claims, **characterized in that** the carrier (2) is designed as elastic textile fabric, in particular as elastic netting.

8. The bandage (1) according to any one of the preceding claims, **characterized in that** the bandage (1) is designed as knee or elbow protector.

9. The bandage (1) according to any one of the preceding claims, **characterized in that** the bandage (1) comprises a ring-shaped elastic band (7) on the edge side.

10. The bandage (1) according to any one of the preceding claims, **characterized in that** the bandage (1) comprises a protector insert, which is surrounded by a padding.

11. The bandage (1) according to any one of the preceding claims, **characterized in that** on a rear side, the carrier (2) is partly designed of a different material, in particular a material, which exerts less pull.

12. The bandage (1) according to any one of the preceding claims, **characterized in that** the strips have a different height, in particular that the height reduces from a center to an edge or that higher and lower strips alternate.

13. The bandage (1) according to any one of the preceding claims, wherein the bandage (1) can be applied around an extremity, in particular a joint, whereby a lower and an upper end of the bandage (1) is defined, **characterized in that** the bandage (1) has a plurality of elastic strips (8a-8d) on a side applied to the extremity, wherein a plurality of lower elastic strips (8a-8d) extend essentially in parallel downwards, starting at a central region (11) and wherein a plurality of upper elastic strips (8a-8d) extend obliquely upwards.

14. The bandage (1) according to the preceding claim, **characterized in that** upper elastic strips (8a-8d) intersect.

15. The bandage (1) according to any one of the preceding claims, **characterized in that** elastic strips (8a-8d) are omitted in the central region (11).

## Revendications

1. Bandage (1), en particulier conçu comme un protecteur (1), le bandage (1) pouvant être appliqué autour d'une extrémité, notamment une articulation, **caractérisé en ce que** le bandage (1) présente une pluralité de bandes élastiques (8a-8d) sur un côté en contact avec l'extrémité, les bandes élastiques (8a-8d) s'étendant latéralement à partir de l'articulation, d'abord latéralement, puis étant déviées vers le bas, et d'autres bandes élastiques (8a-8d) s'étendant vers le haut en oblique vers les côtés, et une zone centrale (11) de forme arrondie étant évidée (11) par les bandes élastiques (8a-8d).

2. Bandage (1) selon la revendication précédente, **caractérisé en ce que** les bandes élastiques (8a-8d) sont apposées (15) sur la face intérieure du bandage (1).

3. Bandage (1) selon l'une des revendications précédentes, **caractérisé en ce que** les bandes élastiques (8a-8d) sont constituées d'un silicone, d'un élastomère ou d'une polyoléfine, notamment d'un polyuréthane.

4. Bandage (1) selon l'une des revendications précédentes, **caractérisé en ce que** les bandes élastiques (8a-8d) sont constituées d'un matériau ayant une dureté selon Shore A (selon DIN ISO 7619-1:2012-2) supérieure à 10, préférablement supérieure à 15, plus préférablement supérieure à 20 et/ou inférieure à 50, préférablement inférieure à 40, plus préférablement inférieure à 35, encore plus préférablement inférieure à 30.

5. Bandage (1) selon l'une des revendications précédentes, **caractérisé en ce que** les bandes élastiques (8a-8d) présentent des interruptions (14), en particulier à une distance de 0,5 à 5 cm
et/ou **en ce que** les autres bandes élastiques (8a-8d) se coupent partiellement.

6. Bandage (1) selon l'une des revendications précédentes, **caractérisé en ce que** le bandage (1) présente au moins par sections 3 à 15, préférablement 6 à 10, bandes élastiques (8a-8d) juxtaposées
et/ou **en ce que** les bandes élastiques (8a-8d) ont une largeur de 0,5 à 2 cm, préférablement de 0,8 à 1,8 cm,
et/ou **en ce que** les bandes élastiques (8a-8d) ont une hauteur de 0,05 à 3 mm, préférablement de 0,1 à 1 mm, plus préférablement de 0,4 à 0,8 mm.

7. Bandage (1) selon l'une des revendications précédentes, **caractérisé en ce que** le support (2) est conçu sous la forme d'un produit textile plat élastique, en particulier d'un tissu réticulé élastique.

8. Bandage (1) selon l'une des revendications précédentes, **caractérisé en ce que** le bandage (1) est conçu comme un protecteur de genou ou de coude.

9. Bandage (1) selon l'une des revendications précédentes, **caractérisé en ce que** le bandage (1) comprend une bande élastique annulaire (7) côté bord.

10. Bandage (1) selon l'une des revendications précédentes, **caractérisé en ce que** le bandage (1) comprend un insert de protection qui est entouré d'un rembourrage.

11. Bandage (1) selon l'une des revendications précédentes, **caractérisé en ce que** le support (2) est conçu sur une face arrière, par zones, dans un autre matériau, en particulier un matériau qui exerce moins de traction.

12. Bandage (1) selon l'une des revendications précédentes, **caractérisé en ce que** les bandes ont une hauteur différente, en particulier **en ce que** la hauteur diminue d'un centre à un bord ou **en ce que** des bandes plus hautes et plus basses alternent.

13. Bandage (1) selon l'une des revendications précédentes, le bandage (1) pouvant être appliqué autour d'une extrémité, notamment une articulation, définissant ainsi une extrémité inférieure et une extrémité supérieure du bandage (1), **caractérisé en ce que** le bandage (1) présente une pluralité de bandes élastiques (8a-8d) sur un côté en contact avec l'extrémité, une pluralité de bandes élastiques inférieures (8a-8d) s'étendant sensiblement parallèlement vers le bas à partir d'une zone centrale (11), et une pluralité de bandes élastiques supérieures (8a-8d) s'étendant obliquement vers le haut.

14. Bandage (1) selon la revendication précédente, **caractérisé en ce que** des bandes élastiques supérieures (8a-8d) se croisent.

15. Bandage (1) selon l'une des revendications précédentes, **caractérisé en ce que** la zone centrale (11) est évidée par les bandes élastiques (8a-8d).
